# EUROPÄISCHE PATENTSCHRIFT

(11) **EP 0 358 044 B1**
(45) Veröffentlichungstag und Bekanntmachung des Hinweises auf die Patenterteilung: **26.01.1994**
(21) Anmeldenummer: 89115526.9
(22) Anmeldetag: 23.08.1989
(51) Int. Cl.: C07D 239/47

(54) **Verfahren zum Fällen von Cytosin aus alkalischen Lösungen mit Schwefelsäure**
Process to precipitate cytosin from alkaline solutions, with sulfuric acid
Procédé pour précipiter la cytosine à partir de solutions alcalines, avec l'acide sulfurique

(30) Priorität: 27.08.1988 DE 3829100
(43) Veröffentlichungstag der Anmeldung: 14.03.1990
(73) Patentinhaber: BOEHRINGER INGELHEIM KG, 55216 Ingelheim (DE); BOEHRINGER INGELHEIM INTERNATIONAL GmbH, 55218 Ingelheim am Rhein (DE)
(72) Erfinder: Hilgers, Georg, Dr., D-6507 Ingelheim (DE); Hess, Joachim, Dr., D-6530 Bingen (DE)

(56) Entgegenhaltungen:
- DE-A- 3 434 142
- US-A- 2 892 840

## Beschreibung

Die vorliegende Erfindung betrifft ein verbessertes Verfahren zum Fällen von Cytosin (I). 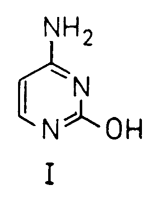
Cytosin (I) kommt als zentralem Zwischenprodukt bei der Darstellung pharmakologisch aktiver Derivate eine große Bedeutung zu. So kann zum Beispiel das Arabinosid des Cytosins wirksam zur Behandlung der Leukämie eingesetzt werden.

Während zum Aufbau des dem Cytosin (I) zugrundeliegenden Pyrimidingerüstes verschiedene Methoden bekannt sind [D.T. Hurst, The Chemistry and Biochemistry of Pyrimidines, Purines and Pteridines, John Wiley and Sons Chichester 1980], ist für die direkte Herstellung des Cytosins (I), welche nicht den vorherigen Aufbau des Pyrimidingrundkörpers notwendig macht, im wesentlichen lediglich die baseninduzierte Umsetzung von Harnstoff mit 3-Ethoxyacrylnitrilen [US-PS 28 92 840; P.J. Tarsio und L. Nicholl, J. Org. Chem. 22 (1957) 192] oder von Harnstoff mit 1-Cyano-2,2-diethoxyethan [A. Bendich, H. Getler und G. B. Brown, J. Biol. Chem. 177 (1949) 565] bekannt.

In den zitierten Verfahren wird Harnstoff (IV) in Gegenwart von Natrium-n-butanolat unter Rückflußbedingungen mit 3-Ethoxyacrylnitril bzw. 1-Cyano-2,2-diethoxyethan zunächst zu einem nicht näher charakterisierten Zwischenprodukt umgesetzt, welches in einem weiteren Reaktionsschritt mit Schwefelsäure behandelt wird. Nachteiligerweise kann bei diesen zum Teil aufwendigen Reaktionen bei diesen Verfahren nicht die freie Cytosin-Base (I) gewonnen werden, sondern es werden lediglich deren Additionssalze mit Schwefelsäure - das Sulfat oder Hemisulfat - isoliert, aus denen das Cytosin (I) selbst nur unter hohen Ausbeuteverlusten gewonnen werden kann.

Das nach dem Auflösen des Cytosinsulfates bzw. -hemisulfates durch Zugabe von Ammoniak isolierte Cytosin (I) ist zudem sehr stark u.a. mit Cytosinsulfat - welches ähnliche Löslichkeitseigenschaften besitzt - verunreinigt.

Eine vollständige Abtrennung des Cytosins (I) von diesen Verunreinigungen ist jedoch nur unter Inkaufnahme von hohen Ausbeuteverlusten an Cytosin (I) und unter Einsatz großer Lösungsmittelmengen möglich.

Weitere Nachteile dieses Verfahrens bestehen in der Notwendigkeit, im Verhältnis zur Ansatzgröße eine sehr große Menge Schwefelsäure einsetzen zu müssen, welche - gemäß dem Stand der Technik - zur Herstellung der Cytosinsalze benötigt wird. Die Cytosinsalze müssen ihrerseits in einem weiteren Reaktionsschritt ebenfalls mit großen Mengen wässeriger Ammoniak-Lösung umgesetzt werden um die Cytosin-Base (I) freizusetzen, woraus zusätzlich Entsorgungsprobleme für die anfallenden Salzmengen resultieren.

Die Abtrennung des als Verunreinigung enthaltenen Cytosinsulfates gelingt nur nach Zugabe ebenfalls relativ großer Mengen von Ethanol.

In der EP 82 339 und der DE-OS 34 34 142 sind Verfahren auf der Basis der Umsetzung von Harnstoff (IV) mit 3-Alkoxyacrylnitrilen und/oder 3,3-Dialkoxypropionitrilen offenbart, welche die Isolierung des Cytosins aus der Reaktionsmischung durch Neutralisation mit Ameisensäure, Essigsäure, Chlorwasserstoff oder Phosphorsäure ermöglichen, ohne die Isolierung eines Zwischenproduktes bzw. eines Cytosinsalzes erforderlich zu machen. Der EP 82 339 und der DE-OS 34 34 142 ist aber auch zu entnehmen, daß Schwefelsäure bei den dort offenbarten Verfahren zur Neutralisation zum Zwecke der Isolierung des freien Cytosins (I) nicht geeignet ist.

Das in der EP 82 339 offenbarte Verfahren liefert Cytosin (I) zudem lediglich in schlechten Ausbeuten, während das Verfahren, welches in der DE-OS 34 34 142 offenbart ist, sehr aufwendig ist und das zweimalige Eindampfen der Reaktionsmischung bis zur Trockne notwendig macht.

Es ist die Aufgabe der vorliegenden Erfindung, ein Verfahren zur Verfügung zu stellen, welches die Herstellung von Cytosin (I) in einem Eintopfverfahren unter Vermeidung des Einsatzes großer Mengen von Lösungs- und Neutralisationsmitteln in hoher Ausbeute und in einem hohen Reinheitsgrad ermöglicht.

Ausgehend von der Umsetzung von 3-Alkoxyacrylnitrilen der allgemeinen Formel II und/oder 3,3-Alkoxypropionitrilen der allgemeinen Formel III mit Harnstoff (IV) in Gegenwart von Alkali- oder Erdalkalialkoholaten bzw -hydroxiden gemäß dem folgendem Reaktionsschema: 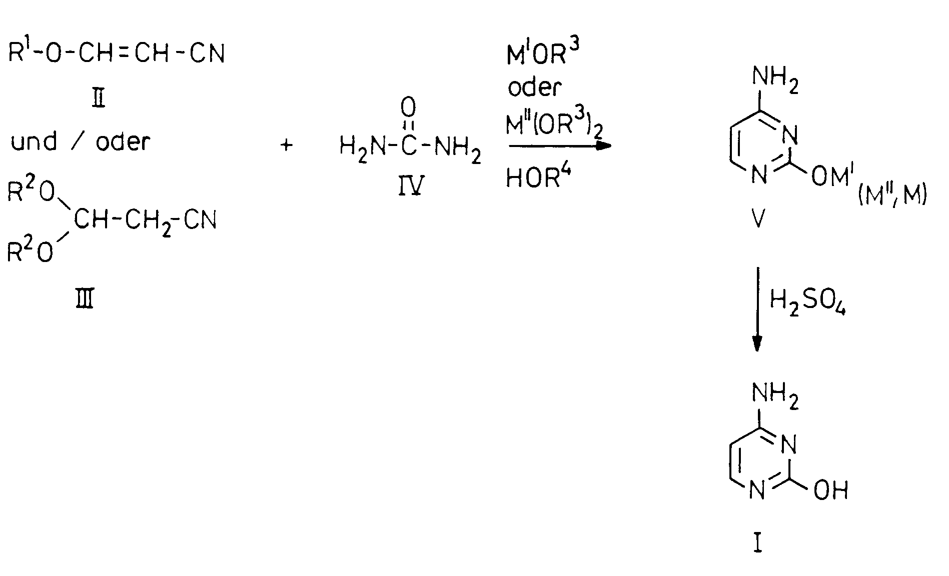
worin
- R¹ und R²: - unabhangig voneinander - einen geradkettigen oder verzweigten Alkylrest oder Alkenylrest mit 1 bis 12 Kohlenstoffatomen, der gegebenenfalls mit einem oder mehreren inerten Substituenten - wie z.B. Fluor, Methoxy, Ethoxy, Amino - untereinander gleich oder verschieden substituiert sein kann, einen ein- oder mehrkernigen Arylrest mit 6 bis 10 Kohlenstoffatomen, wobei der Aromat mit einer oder mehreren Alkylgruppe(n), Alkoxygruppe(n), Nitrogruppe(n), Aminogruppe(n) und/oder einem oder mehreren Halogenatom(en) - untereinander gleich oder verschieden - substituiert sein kann, einen Aralkylrest mit 7 bis 14 Kohlenstoffatomen, wobei der über eine Alkylenkette gebundene Aromat ein- oder mehrkernig sein kann und mit einer oder mehreren Alkylgruppe(n), Alkoxygruppe(n), Nitrogruppe(n), Aminogruppe(n) und/oder einem oder mehreren Halogenatom(en) - untereinander gleich oder verschieden - substituiert sein kann, einen über eine Alkylenkette gebundenen Cycloalkylrest mit 5 bis 9 Kohlenstoffatomen, worin ein oder mehrere Kohlenstoffatom(e) durch ein Heteroatom - wie z.B. Stickstoff, Sauerstoff oder Schwefel - ersetzt sein kann und welcher gegebenenfalls mit einem oder mehreren der oben angegebenen inerten Substituenten untereinander gleich oder verschieden substituiert sein kann;
- R³: Wasserstoff, Methyl, Ethyl, Propyl, Isopropyl, Butyl, Isobutyl, tert.-Butyl, Pentyl, tert.-Pentyl (2-Methyl-2-butyl), Neopentyl, Hexyl, Isohexyl, Cyclohexyl;
- R⁴: Methyl, Ethyl, Propyl, Isopropyl, Butyl, Isobutyl, tert.-Butyl, Pentyl, tert.-Pentyl, Neopentyl, Hexyl, Isohexyl, Cyclohexyl;
- M: ein Kation;
- M^{I}: ein Alkalimetall-Kation;
- M^{II}: ein Erdalkalimetall-Kation;
bedeutet,
erhält man das entsprechende Alkali- bzw. Erdalkalisalz als Cytosins V.

Bevorzugt bedeutet:
- R¹ und R²: - unabhängig voneinander - einen verzweigten oder unverzweigten Alkylrest mit 1 bis 4 Kohlenstoffatomen) - wie z.B. Methyl, Ethyl, Propyl, Isopropyl, Butyl, Isobutyl, tert.-Butyl;
- R³: Methyl, Ethyl, Propyl, Isopropyl, Butyl, tert.-Butyl, tert.-Pentyl (2-Methyl-2-butyl);
- R⁴: Methyl, Ethyl, Propyl, Isopropyl, Butyl, tert.-Butyl, Pentyl, tert.-Pentyl;
- M^{I}: Na⁺, K⁺.

Besonders bevorzugt bedeutet
- R¹ und R²: - unabhängig voneinander - Methyl, Ethyl;
- R³: Methyl, Ethyl, Isopropyl, tert.-Butyl, tert.-Pentyl;
- R⁴: Ethyl, Propyl, Isopropyl, Butyl, tert.-Butyl;
- M^{I}: Na⁺, K⁺.

Überraschenderweise wurde nun gefunden, daß reines Cytosin (I) durch Neutralisieren bzw. Ansäuern einer wässerigen Lösung der aus dieser Umsetzung resultierenden Reaktionsmischung mit Schwefelsäure erhalten werden kann. Hierzu wird erfindungsgemäß die wässerige basische Lösung der obengenannten alkalischen Reaktionsmischung bei erhöhter Temperatur mit Schwefelsäure auf einen pH-Wert im Bereich von 2-7, vorzugsweise 6-7 eingestellt. Anschließend werden die festen Bestandteile von der Reaktionslösung - beispielsweise auf dem Wege der Filtration - abgetrennt und das beim Abkühlen des Filtrates ausfallende Cytosin isoliert.

Das nach dem erfindungsgemäßen Verfahren hergestellte Cytosin (I) ist weder durch Cytosinsulfat noch durch Cytosinhemisulfat verunreinigt und fällt in hohen Ausbeuten und in einer sehr hohen Reinheit an, was die direkte Weiterverarbeitung des Produktes - insbesondere zur Darstellung von Pharmazeutika - ohne weitere Reinigungsschritte erlaubt.

Die Darstellung des Alkali- oder Erdalkalisalzes V des Cytosins (I) erfolgt - auf an und für sich bekannte Weise - durch Umsetzung von 3-Alkoxyacrylnitrilen bzw. 3,3-Dialkoxypropionitrilen - bevorzugt 3-Methoxyacrylnitril bzw. 3-Ethoxyacrylnitril - mit Harnstoff (IV) und einer Base - vorzugsweise einem Alkalialkoholat und besonders bevorzugt Natrium-tert.-amylat, Natrium-tert.-butylat, Natriumisopropylat, Natriumethylat oder Natriummethylat - in einem Lösungsmittel.

Als Lösungsmittel finden polare Solvenzien wie z.B. Alkohole - vorzugsweise C₁-C₆-Alkohole - und besonders bevorzugt Ethanol, n-Propanol, Isopropanol, n-Butanol, tert.-Butanol Verwendung.

Zum Fällen des Cytosins (I) wird Schwefelsäure, bevorzugt verdünnte und besonders bevorzugt 50 proz. Schwefelsäure verwandt.

Die Fällung des Cytosins (I) kann aber auch in der Weise erfolgen, daß der pH-Wert der Reaktionslösung mit einer Mineralsäure oder Carbonsäure zunächst erniedrigt wird und die eigentliche Fällung des Cytosins (I) mit Schwefelsäure durchgeführt wird, wobei das so hergestellte Cytosin (I) ebenfalls in hoher Ausbeute und in einem hohen Reinheitsgrad isoliert wird.

Die Umsetzung des Harnstoffs (IV) mit 3-Alkoxyacrylnitrilen bzw. 3,3-Dialkoxypropionitrilen - bevorzugt mit 3-Methoxyacrylnitril oder mit 3-Ethoxyacrylnitril bzw. mit 3,3-Dimethoxypropionitril - und - vorzugsweise - dem Alkalialkoholat wird zweckmäßigerweise unter wasserfreien Bedingungen und unter Inertgas in einem Temperaturbereich zwischen 70 und 80°C und vorzugsweise bei 75°C durchgeführt. - Je nach Reaktivität der Reaktanden kann jedoch auch eine höhere oder niedrigere Temperaturführung notwendig sein.

Bei der Durchführung hat es sicht als zweckmäßig erwiesen, den Harnstoff (IV) zu der unter Inertgas auf 75°C erwärmten Lösung des Alkalialkoholates bzw. Erdalkalialkoholates in einem polaren Lösungsmittel - vorzugsweise einem Alkohol - zuzufügen, wobei bei der Verwendung von Alkoholen als Reaktionsmedium der Alkoholatrest der Base (R³) nicht notwendigerweise mit dem Alkylrest (R⁴) des entsprechenden Alkohols, der als Lösungsmittel eingesetzt wird, identisch sein muß.

Darauf wird das 3-Alkoxyacrylnitril bzw. 3,3-Dialkoxypropionitril - bevorzugt 3-Methoxy- oder 3-Ethoxyacrylnitril bzw. 3,3-Dimethoxypropionitril - gewünschtenfalls in einer Lösung und gegebenenfalls in dem als Reaktionsmedium eingesetzten Lösungsmittel - zugefügt, wonach die Reaktionsmischung auf Rückflußtemperatur erwärmt wird. Nach Beendigung der Reaktion wird das Reaktionsgemisch - gegebenenfalls unter vermindertem Druck - vom größten Teil des Lösungsmittels befreit, bis eine noch rührfähige Suspension verbleibt.

Danach wird die Suspension mit Wasser versetzt und alle Reaktionsbestandteile mit einem Siedepunkt < 85°C werden abdestilliert.

Im Anschluß daran wird bei einer Temperatur im Bereich von 50 bis 80°C vorzugsweise in einem Temperaturintervall von 70 - 80°C mit Schwefelsäure, bevorzugt mit verdünnter Schwefelsäure und besonders bevorzugt mit ca. 50 proz. Schwefelsäure der pH-Wert des Reaktionsgemisches auf einen Wert im Bereich von 2-7 vorzugsweise 6-7 eingestellt, die Lösung mit einer Suspension von Aktivkohle in Wasser versetzt und auf Rückflußtemperatur erwärmt.

Die Aktivkohle wird anschließend mittels eines vorgeheizten Druckfilters von dem noch ca. 80°C warmen Reaktionsgemisch getrennt und das Filtrat auf 20°C abgekühlt. Das dabei ausfallende Cytosin (I) wird isoliert und mit Wasser gewaschen und i. Vak. bei 60°C bis zur Gewichtskonstanz getrocknet.

Pro mol 3-Alkoxyacrylnitril - bevorzugt 3-Methoxyacrylnitril oder 3-Ethoxyacrylnitril - oder 3,3-Dialkoxypropionitril - bevorzugt 3,3-Dimethoxypropionitril - werden 1 bis 3, vorzugsweise 1 bis 2 mol Harnstoff und 1 bis 3 mol und bevorzugt 1,7 bis 2,2 mol Base bzw. Alkalialkoholat eingesetzt. Bei der Verwendung von wasserhaltigen Lösungsmitteln ist es zweckmäßig, die Menge der eingesetzten Base entsprechend zu erhöhen.

### Beispiel

9,5 l Isopropanol werden unter Inertgasatmosphäre mit 2,085 kg (37,819 mol) Natriummethylat (Gehalt 98 %) versetzt und auf 75°C erwärmt. Im Anschluß daran werden unter Rühren 2,107 kg (34,380 mol) Harnstoff (Gehalt 98 %) bei 75°C über einen Zeitraum von 20 min. eingetragen. Nach beendeter Zugabe wird eine Mischung von 2,000 kg (20,181 mol) 3-Ethoxyacrylnitril (Gehalt 98 %) ebenfalls unter Rühren und bei 75°C über einen Zeitraum von 15 Minuten zudosiert. Die Dosieranlage wird mit 1 l Isopropanol gespült.

Die braune Reaktionsmischung wird 3 Stunden lang auf Rückflußtemperatur erwärmt, wobei die Bildung eines Feststoffes zu beobachten ist. Anschließend werden 9,4 l Isopropanol abdestilliert. Die verbleibende Suspension wird unter Rühren innerhalb von 10 Minuten mit 11,5 l Wasser versetzt. Die entstandene braune Lösung wird auf Rückflußtemperatur erwärmt (ca. 85°C) und es werden 3,0 l Isopropanol/Wasser-Gemisch abdestilliert. Danach läßt man die Reaktionsmischung auf 75°C abkühlen und neutralisiert mit 50 proz. Schwefelsäure bis ein pH-Wert von 7 erreicht ist, fügt 2 l Wasser zu und läßt die Reaktionsmischung auf 60°C abkühlen. Im Anschluß daran trägt man eine Dispersion von 0,125 kg Aktivkohle in 0,5 l Wasser in das Gemisch ein, erwärmt unter Rühren auf Rückflußtemperatur und läßt 15 Minuten lang nachrühren. Die Reaktionsmischung wird anschließend über einen auf ca. 80°C aufgeheizten Druckfilter filtriert und der Rückstand mit 2 l auf 80°C erwärmten Wasser gewaschen. Das Filtrat wird auf 20°C abgekühlt und das auskristallierte Produkt über eine mit einem Filtertuch belegte Nutsche abgesaugt. Der Rückstand wird mit Wasser (20°C) gewaschen und trockengesaugt und im Trockenschrank unter vermindertem Druck bei einer Temperatur von 60°C bis zur Gewichtskonstanz getrocknet. Es werden 1,75 kg (78,1 % d. Th.) Cytosin als weißes amorphes Pulver erhalten.

Schmp.: 313-316°C Zers.
Reinheit:> 99% (HPLC)

## Patentansprüche

1. Verfahren zum Fällen von Cytosin, dadurch gekennzeichnet, daß man den pH-Wert einer wässerigen Lösung eines Cytosinsalzes V 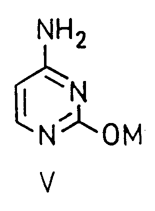 mit Schwefelsäure in einem Temperaturintervall von 70 bis 80°C auf einen Wert im Bereich von 2 - 7 einstellt, die erhitzte Reaktionslösung von festen Bestandteilen befreit und das beim Abkühlen der Reaktionslösung auf 20°C ausfallende Cytosin isoliert.

2. Verfahren zum Fällen von Cytosin nach Anspruch 1, dadurch gekennzeichnet, daß man ein Erdalkali- oder Alkalisalz des Cytosins einsetzt.

3. Verfahren zum Fällen von Cytosin nach Anspruch 1 oder 2, dadurch gekennzeichnet, daß man ein Alkalisalz des Cytosins einsetzt.

4. Verfahren zum Fällen von Cytosin nach einem der Ansprüche 1 bis 3, dadurch gekennzeichnet, daß man das Natrium- oder Kaliumsalz des Cytosins einsetzt.

5. Verfahren zum Fällen von Cytosin, dadurch gekennzeichnet, daß man den pH-Wert einer wässerigen Lösung eines Cytosinsalzes V mit einer Mineralsäure oder einer organischen Säure erniedrigt und das Cytosin gemäß Anspruch 1 mit Schwefelsäure fällt.

6. Verfahren zum Fällen von Cytosin nach Anspruch 5, dadurch gekennzeichnet, daß man ein Erdalkali- oder Alkalisalz des Cytosins einsetzt.

7. Verfahren zum Fällen von Cytosin nach Anspruch 5 oder 6, dadurch gekennzeichnet, daß man ein Alkalisalz des Cytosins einsetzt.

8. Verfahren zum Fällen von Cytosin nach einem der Ansprüche 5 bis 7, dadurch gekennzeichnet, daß man das Natrium- oder Kaliumsalz des Cytosins einsetzt.

9. Verfahren zum Fällen von Cytosin nach einem der Ansprüche 1 bis 8, dadurch gekennzeichnet, daß man 50 proz. Schwefelsäure einsetzt.

## Claims

1. Process for precipitating cytosine, characterised in that the pH of an aqueous solution of a cytosine salt V 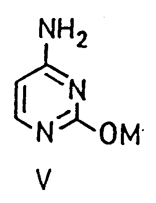 is adjusted with sulphuric acid at a temperature in the range from 70 to 80°C to a level in the range from 2 to 7, the heated reaction solution is freed from solid constituents and the cytosine precipitated as the reaction solution cools is isolated.

2. Process for precipitating cytosine according to claim 1, characterised in that an alkaline earth or alkali metal salt of cytosine is used.

3. Process for precipitating cytosine according to claim 1 or 2, characterised in that an alkali metal salt of cytosine is used.

4. Process for precipitating cytosine according to one of claims 1 to 3, characterised in that the sodium or potassium salt of cytosine is used.

5. Process for precipitating cytosine, characterised in that the pH of an aqueous solution of a cytosine salt V is lowered using an inorganic or organic acid and the cytosine is precipitated with sulphuric acid according to claim 1.

6. Process for precipitating cytosine according to claim 5, characterised in that an alkaline earth or alkali metal salt of cytosine is used.

7. Process for precipitating cytosine according to claim 5 or 6, characterised in that an alkali metal salt of cytosine is used.

8. Process for precipitating cytosine according to one of claims 5 to 7, characterised in that the sodium or potassium salt of cytosine is used.

9. Process for precipitating cytosine according to one of claims 1 to 8, characterised in that 50% sulphuric acid is used.

## Revendications

1. Procédé pour précipiter la cytosine, caractérisé en ce que l'on ajuste à une valeur située dans le domaine de 2 à 7 le pH d'une solution aqueuse d'un sel de cytosine V 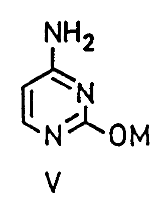 avec l'acide sulfurique dans un intervalle de température de 70 à 80°C, on débarrasse la solution réactionnelle chauffée des constituants solides et on isole la cytosine qui précipite lors du refroidissement à 20°C de la solution réactionnelle.

2. Procédé pour précipiter la cytosine selon la revendication 1, caractérisé en ce que l'on utilise un sel alcalinoterreux ou alcalin de la cytosine.

3. Procédé pour précipiter la cytosine selon la revendication 1 ou 2, caractérisé en ce que l'on utilise un sel alcalin de la cytosine.

4. Procédé pour précipiter la cytosine selon l'une des revendications 1 à 3, caractérisé en ce que l'on utilise le sel de sodium ou de potassium de la cytosine.

5. Procédé pour précipiter la cytosine, caractérisé en ce que l'on abaisse le pH d'une solution aqueuse d'un sel de cytosine V avec un acide minéral ou un acide organique et l'on précipite la cytosine selon la revendication 1 avec l'acide sulfurique.

6. Procédé pour précipiter la cytosine selon la revendication 5, caractérisé en ce que l'on utilise un sel alcalinoterreux ou alcalin de la cytosine.

7. Procédé pour précipiter la cytosine selon la revendication 5 ou 6, caractérisé en ce que l'on utilise un sel alcalin de la cytosine.

8. Procédé pour précipiter la cytosine selon l'une des revendications 5 à 7, caractérisé en ce que l'on utilise le sel de sodium ou de potassium de la cytosine.

9. Procédé pour précipiter la cytosine selon l'une des revendications 1 à 8, caractérisé en ce que l'on utilise de l'acide sulfurique à 50%.
